# FASCICULE DE BREVET EUROPEEN

(11) **EP 2 221 026 B1**
(45) Date de publication et mention de la délivrance du brevet: **20.03.2013**
(21) Numéro de dépôt: 10003893.4
(22) Date de dépôt: 12.04.2006
(51) Int. Cl.: A61F 2/42

(54) **Dispositif d'osteosynthese intramedullaire de deux parties d'os, notamment de la main et/ou du pied**
Vorrichtung für die intramedulläre Osteosynthese von zwei Knochenteilen, insbesondere der Hand und/oder des Fußes
Device for intramedullary osteosynthesis of two parts of bone, in particular of the hand and/or foot

(30) Priorité: 14.04.2005 FR 0550957
(43) Date de publication de la demande: 25.08.2010
(62) Demande divisionnaire de: 06743805.1
(73) Titulaire: Memometal Technologies, société anonyme, 35170 Bruz (FR); Augoyard, Marc, 69160 Tassin la Demi Lune (FR); Peyrot, Jacques, 69160 Tassin la Demi Lune (FR); Meusnier, Tristan, 42000 St Etienne (FR)
(72) Inventeur: Prandi, Bernard, 35700 Rennes (FR)
(74) Mandataire: Benech, Frédéric

(56) Documents cités:
- FR-A1- 2 846 545
- US-A- 5 425 777

## Description

L'invention se rattache au secteur technique des implants orthopédiques, notamment pour arthrodèses et ostéosynthèses.

On rappelle que le but d'une arthrodèse est d'obtenir une très bonne stabilité aussi bien primaire que secondaire et de mettre, ou de maintenir, en compression deux parties d'os ou fragments osseux qu'il convient d'immobiliser. La stabilité est critique pour obtenir la consolidation, tout en minimisant les problèmes annexes tels que douleur, gonflement... L'effet de compression permet de consolider plus rapidement l'ostéotomie dans la position choisie par le chirurgien lors de l'opération.

Différentes solutions techniques ont été proposées pour réaliser une arthrodèse, notamment au niveau du pied, de la main, du poignet, ... On peut citer, par exemple, les agrafes basiques sans mémoire de forme qui n'assurent pas une compression, à l'inverse des agrafes à mémoire qui permettent de mettre en compression les deux parties d'os à consolider, ce qui correspond au but recherché.

Toutefois, pour obtenir une stabilité satisfaisante, il est nécessaire de mettre deux, voire trois agrafes, dans des plans différents. Il en résulte un encombrement important, ce qui limite les applications (articulation métacarpo-phalangienne par exemple). On a proposé également des plaques et vis extramédullaires qui nécessitent un encombrement relativement important. Il est à cet égard difficilement envisageable de les miniaturiser, ce qui pourrait générer des problèmes de tenue et de rigidité. Certains types de vis peuvent être utilisées en intramédullaire, mais génèrent des difficultés de positionnement (passage par la pulpe notamment).

On peut également utiliser des broches qui présentent un encombrement réduit. Toutefois, la stabilité obtenue n'est pas satisfaisante et il est nécessaire de procéder à un retrait.

On connaît également (FR 2 846 545) des dispositifs comprenant des pattes déformables par mémoire de forme reliées par une barre centrale. De tels dispositifs présentent des inconvénients au niveau de la jonction entre les parties d'os.

Enfin, on connaît des clous intramédullaires mais ces derniers nécessitent un agrafage complémentaire afin de les bloquer en rotation.

L'invention s'est fixée pour but de remédier à ces inconvénients d'une manière simple, sûre, efficace et rationnelle.

Le problème que se propose de résoudre l'intention est de permettre la fixation de deux parties d'os l'une à l'autre, d'une manière rigide avec une compression dynamique et rétentive afin d'obtenir une ostéosynthèse fiable et rapide.

Pour résoudre un tel problème, il a été conçu et mis au point un élément d'arthrodèse intramédullaire qui est constitué par un corps de forme allongée présentant, successivement, à partir de l'une de ses extrémités, une zone d'ancrage coopérant avec l'une des parties d'os à immobiliser, une zone médiane apte à résister aux contraintes de cisaillement et de flexion, et une zone d'ancrage dans l'autre partie d'os à immobiliser, chacune desdites zones d'ancrage est profilée et réalisée dans un matériau sélectionné pour permettre une introduction dans les parties d'os sans abord pulpaire, puis pour assurer un ancrage dans lesdites parties d'os en évitant tout mouvement de rotation, en résistant à la traction et en maintenant un effort de compression.

L'invention trouve une application particulièrement avantageuse, qui ne saurait toutefois être considérée comme limitative, pour la réalisation d'arthrodèses au niveau des phalanges proximales et moyennes, pour les articulations interphalangiennes proximales et les articulations interphalangiennes distales, au niveau de la main et/ou au niveau du pied.

Pour résoudre le problème posé de tenir compte de l'anatomie, et notamment du rétrécissement interne de l'os, la zone médiane est reliée à au moins l'une de zones d'ancrage par une zone de liaison.

Pour résoudre le problème posé de permettre l'implantation de l'élément puis la mise en compression des fragments osseux, les zones d'ancrage sont réalisées dans un matériau à mémoire de forme pour être déformées par effet thermique et/ou mécanique.

Pour réaliser les zones d'ancrage, qui peuvent être identiques ou non, différentes solutions techniques sont possibles, en fonction notamment du type d'arthrodèse réalisée et des articulations à traiter. Par exemple : l'une des zones d'ancrage présente deux pattes ou ailettes aptes à être écartée sous l'effet de la mémoire de forme ; l'une des zones d'ancrage présente une patte ou tige apte à être recourbée sous l'effet de la mémoire de forme; l'une des zones d'ancrage présente, dans son épaisseur, une lumière pour permettre une déformation par élasticité, ou mémoire, sous l'effet de la mémoire de forme.

Dans une forme de réalisation, l'ensemble du corps a une section transversale méplate.

L'invention est exposée ci-après plus en détail à l'aide des figures des dessins annexés dans lesquels
- la figure 1 est une vue en plan à caractère schématique montrant la mise en place de l'élément d'arthrodèse intramédullaire selon l'invention entre une phalange proximale et une phalange moyenne pour assurer le blocage de l'articulation interphalangienne proximale ;
- la figure 2 est une vue en plan d'un exemple de réalisation de l'élément d'arthrodèse au moment de son introduction ;
- la figure 3 est une vue correspondant à la figure 2 montrant l'élément d'arthrodèse après son implantation pour réaliser la compression ;

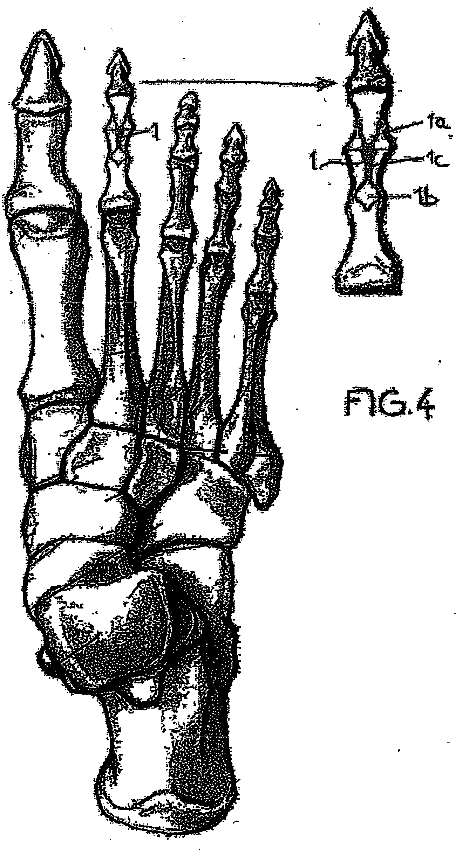

- la figure 4 montre la mise en place de l'élément selon l'invention au niveau d'un orteil.

L'élément d'arthrodèse selon l'invention est constitué par un corps de forme allongée désigné dans son ensemble par (1). Chacune des extrémités du corps est conformée pour réaliser une zone d'ancrage (1a) à une zone d'ancrage (1b). Entre les deux zones d'ancrage (1a) et (1b), est formée au moins une zone médiane (1c) apte à résister aux contraintes de cisaillement et de flexion. Généralement, les contraintes de cisaillement et de flexion s'exercent au niveau du foyer osseux à consolider. La forme de cette zone médiane (1c) est adaptée à la forme interne de l'os. Sa longueur est déterminée afin d'autoriser un léger décalage dans le centrage.

A titre indicatif nullement limitatif, cette zone médiane peut avoir une section rectangulaire de 2 à 3 mm x 1 à 1, 5 mm environ et une longueur de 3 à 5 mm environ (pour le pied et la main).

Les zones d'ancrage (1a) et (1b) sont conformées pour éviter tout mouvement de rotation, résister à une sollicitation en traction, et maintenir la compression manuelle mise au moment de la pose par le chirurgien afin de réduire le foyer. Pour obtenir ce résultat, les zones d'ancrage (1a) et (1b) sont réalisées dans un matériau à mémoire de forme pour être déformées par effet thermique (mémoire tiède) ou par effet mécanique (superélasticité). Le but recherché, au niveau des zones d'ancrage, en considérant, d'une part, leur profil et, d'autre part, la nature du matériau les constituant est de permettre une introduction dans les parties d'os, notamment par voie dorsale sans abord pulpaire, d'une part, puis d'assurer un ancrage dans ladite partie d'os afin d'obtenir ou de maintenir l'effort de compression recherché, d'autre part. Les zones d'ancrage (1a) et (1b) sont identiques ou non, selon le type d'os et sa morphologie. En fonction du type d'arthrodèse réalisée, c'est-à-dire de la nature des articulations interphalangiennes à bloquer par exemple, les zones d'ancrage (1a) et (1b) peuvent présenter différentes formes de réalisation.

Par exemple, l'une des zones d'ancrage (1a) présente deux pattes ou ailettes aptes à être écartées sous un effet thermique par exemple. Ou bien, ces zones d'ancrage (1a) peuvent présenter une seule patte ou tige apte à être recourbée sous l'effet de la mémoire du matériau la constituant. Ou bien, la zone d'ancrage (1b) présente, dans son épaisseur, une lumière pour permettre une déformation par élasticité, sous un effet thermique par exemple, et maintenir la position par une pression sur la longueur de l'os.

Suivant une autre caractéristique de l'invention, pour tenir compte de l'anatomie des différentes phalanges par exemple, à savoir le rétrécissement interne de l'os (forme sablier), la zone médiane (1c) est reliée à au moins l'une des zones d'ancrage (1b) par une zone de liaison (1d) plus fine.

On renvoie aux figures des dessins qui montrent un exemple de réalisation d'un élément d'arthrodèse intramédullaire.

Dans cet exemple de réalisation, le corps (1) présente, à l'une de ses extrémités, une zone d'ancrage (la) sous forme de deux pattes ou ailettes (1a1) - (1a2). Cette zone d'ancrage (1a) est prolongée par la zone médiane (1c) de forme générale, vue en plan, sensiblement triangulaire. La zone médiane (1c) est raccordée à l'autre zone d'ancrage d'extrémité (1b) par une zone de liaison (1d) de forme générale, vue en plan, sensiblement rectangulaire. La zone d'ancrage (1b) présente, dans son épaisseur, une lumière de forme générale sensiblement oblongue (1b1).

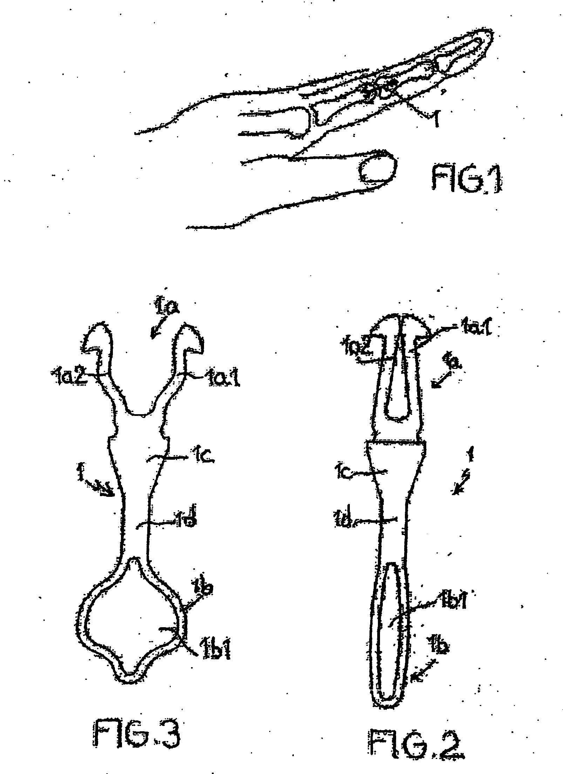

On renvoie à la figure 2 qui montre l'élément au moment de son introduction, c'est-à-dire avant écartement des pattes (1a1) - (la2), et ouverture de la lumière (1b1). Par exemple, cette configuration est obtenue lorsque l'ensemble de l'élément est soumis à une basse température très inférieure à celle du corps humain par exemple. Inversement, après implantation (figure 3), sous l'effet de la chaleur du corps, les pattes (1a1) et (1a2) sont écartées, de même que la lumière (1b1) ce qui provoque, d'une manière concomitante, une déformation de la zone d'ancrage (1b).

A noter que le profil de la zone médiane (1c) évite l'enfoncement quand on vient refermer le foyer.

Dans une forme de réalisation en variante, la zone de liaison (1d) peut être fendue pour bénéficier d'un effet de gonflement par mémoire de forme et de renforcement de l'ancrage dans la zone diaphysaire.

On rappelle que l'élément selon l'invention est particulièrement bien adapté pour le traitement de la pathologie "orteil en griffe ou en marteau" en réalisant une arthrodèse au niveau des phalanges P1 et P2 sur les rayons 2 à 5, en observant que de telles applications ne doivent pas être considérées comme limitatives, moyennant des adaptations essentiellement dimensionnelles (réimplantations de doigt, arthrodèse de l'articulation interphalangienne distale et de l'articulation interphalangienne proximale de la main, arthrodèse MP du gros orteil).

Bien évidemment, l'ensemble de l'élément d'arthrodèse selon l'invention peut présenter des caractéristiques de construction aptes à améliorer notamment l'ancrage et la compression.

Par exemple :
- des crans sur les pattes d'un des côtés pour un meilleur ancrage dans l'os spongieux ;
- des pattes ondulées implantées (droites avant implantation) pour permettre un raccourcissement et donc une mise en compression du foyer d'arthrodèse supplémentaire par rapport à un simple maintien ;
- une zone centrale conique pour éviter un enfoncement non désiré de l'implant au moment où l'on vient fermer le foyer.

A titre indicatif, la mémoire utilisée est préférentiellement de la mémoire tiède, de sorte qu'un chauffage n'est pas nécessaire car il n'y a pas d'accès. Le début d'ouverture s'effectue aux environs de 15 à 20°C, tandis que la fin s'effectue aux environs de 30 à 35°C.

La technique opératoire demeure classique.

## Revendications

1. Dispositif d'ostéosynthèse intra médullaire de deux parties d'os, notamment de la main ou du pied, constitué d'un corps de forme allongée présentant, successivement, à partir de l'une de ses extrémités, une zone d'ancrage (1a) coopérant avec l'une des partie d'os à immobiliser, une zone médiane (1c) apte à résister aux contraintes de cisaillement et de flexion, et une zone d'ancrage (1b) dans l'autre partie d'os à immobiliser,
chacune desdites zones d'ancrage (1a) et (1b) étant réalisée dans un matériau à mémoire de forme apte à permettre leur déformation par effect thermique ou mécanique, afin de permettre une introduction dans la partie d'os sans abord pulpaire, puis assurer un ancrage en évitant tout mouvement de rotation, en résistant à la traction et en maintenant un effort de compression,
une des zonas d'ancrage (1a) présentant deux pattes ou ailettes (1a1) et (1a2) aptes à être écartées sous l'effet de la mémoire de forme, **caractérisé en ce que** le zone médiane (1e) est profilée pour éviter l'enfoncement quant on vient refermer le foyer de fracture et est reliée à la zone d'ancrage (1b) par une zone de liaison (1d) de section réduite.

2. Dispositif selon la revendication 1, **caractérisé en ce que** l'autre zone d'ancrage présente de façons identique deux pattes ou ailettes aptes à être écartées sous l'effet de la mémoire de forme.

3. dispositif selon la revendication 1, **caractérisé en ce que** l'autre zone d'ancrage présente dans son épaisseur, une lumière (1b1) pour permettre une déformation par élasticité sous l'effet de la mémoire de forme.

4. Dispositif selon l'une quelconque des revendications précédentes, **caractérisé en ce que** le corps est de section transversale méplate.

5. Dispositif selon l'une quelconque des revendications précédentes, **caractérisé en ce que** l'une des zones d'ancrage (1a) présente une patte ou tige apte à être recourbée sous l'effet de la mémoire de forme.

6. Dispositif selon l'une quelconque des revendications précédentes **caractérisé en ce que** la zone médiane (1c) est de forme générale, vue en plan, sensiblement triangulaire.

7. Dispositif selon l'une quelconque des revendications précédentes, **caractérisé en ce que** la longueur de la zone médiane (1) est comprise encre 3 mm et 5 mm, la rendant propre à autoriser un léger décalage dans le centrage du dispositif.

8. Dispositif selon l'une quelconque des revendications précédentes, **caractérisé en ce que** la zone de liaison (1d) est de forme générale, vue en plan, sensiblement rectangulaire.

9. Dispositif selon l'une quelconque des revendications précédentes, **caractérise en ce que** la zone de liaison (1d) est fendue pour bénéficier de l'effet de ronflement par mémoire de forme et de renforcement de l'ancrage dans la zone diaphysaire.

10. Dispositif selon l'une quelconque des revendications précédentes, **caractérisé en ce que** les pattes comportent des crans sur l'un de leur cotés.

11. Dispositif selon l'une quelconque des revendications précédentes, **caractérisé en ce que** les pattes sont droites avant implantation et agencées pour être ondulées après la mise en oeuvre de la mémoire de forme pour permettre un racccourcissement.

## Claims

1. Device for intramedullar osteosynthesis of two bone parts, in particular of the hand or of the foot, constituted by a body of elongated shape having successively, from one of its ends, an anchoring zone (1a) co-operating with one of the bone parts to be immobilised, a median zone (1c) capable of withstanding the shearing and bending stresses, and an anchoring zone (1b) for anchoring in the other bone part to be immobilised,
each of said anchoring zones (1a) and (1b) being made of a material with a shape memory capable of permitting their deformation by thermal or mechanical effect, in order to allow introduction into the bone part without an approach via the pulp, then to provide an anchorage while preventing any rotational movement, resisting traction and maintaining a compressive load,
one of the anchoring zones (1a) having two legs or tabs (1a1) and (1a2) capable of being moved apart under the effect of the shape memory, **characterised in that** the median zone (1c) is profiled to avoid sinking when closing the fracture site and is connected to the anchoring zone (1b) by a linking zone (1d) of reduced section.

2. Device according to claim 1, **characterised in that** the other anchoring zone has in identical fashion two legs or tabs capable of being moved apart under the effect of the shape memory.

3. Device according to claim 1, **characterised in that** the other anchoring zone has in its thickness an opening (1b1) to permit deformation through elasticity under the effect of the shape memory.

4. Device according to any one of the preceding claims, **characterised in that** the body is of flat transverse section.

5. Device according to any one of the preceding claims, **characterised in that** one of the anchoring zones (1a) has a leg or limb capable of being curved under the effect of the shape memory.

6. Device according to any one of the preceding claims, **characterised in that** the median zone (1c) is substantially triangular in general shape, in plan view.

7. Device according to any one of the preceding claims, **characterised in that** the length of the median zone (1c) lies between 3 mm and 5 mm, making it suitable to permit a slight displacement in the centring of the device.

8. Device according to any one of the preceding claims, **characterised in that** the linking zone (1d) is of substantially rectangular general shape, in plan view.

9. Device according to any one of the preceding claims, **characterised in that** the linking zone (1d) is split in order to benefit from the effect of swelling through the shape memory and reinforcement of the anchorage in the diaphyseal zone.

10. Device according to any one of the preceding claims, **characterised in that** the legs comprise notches on one of their sides.

11. Device according to any one of the preceding claims, **characterised in that** the legs are straight before implantation and arranged to be undulating after implementation of the shape memory to allow shortening.

## Patentansprüche

1. Vorrichtung für die intramedulläre Osteosynthese von zwei Knochenteilen, insbesondere der Hand oder des Fußes, bestehend aus einem länglich geformten Körper, der von einem seiner Enden ausgehend aufeinander folgend eine Verankerungszone (1a), die mit einem der zu fixierenden Knochenteile zusammenwirkt, eine mittige Zone (1c), die geeignet ist, den Biege- und Scherbeanspruchungen standzuhalten, und eine Zone (1b) zur Verankerung in dem anderen zu fixierenden Knochenteil aufweist,
wobei jede der Verankerungszonen (1a) und (1b) aus einem Material mit Formgedächtnis hergestellt ist, das geeignet ist, ihre Verformung durch thermische oder mechanische Wirkung zu ermöglichen, um eine Einführung in den knochenmarkfreien Knochenteil zu ermöglichen und anschließend eine Verankerung sicherzustellen, wobei jede Drehbewegung vermieden wird, dem Zug standgehalten wird und eine Kompressionskraft aufrechterhalten bleibt,
wobei eine der Verankerungszonen (1a) zwei Schenkel bzw. Flügel (Ia1) und (Ia2) aufweist, die geeignet sind, unter der Wirkung des Formgedächtnisses gespreizt zu werden,
**dadurch gekennzeichnet,**
**dass** die mittige Zone (1c) profiliert ist, um ein Eindrücken zu vermeiden, wenn der Frakturherd wieder verschlossen wird, und über eine Verbindungszone (1d) mit verringertem Querschnitt mit der Verankerungszone (1 b) verbunden ist.

2. Vorrichtung nach Anspruch 1,
**dadurch gekennzeichnet,**
**dass** die andere Verankerungszone gleichermaßen zwei Schenkel bzw. Flügel aufweist, die geeignet sind, unter der Wirkung des Formgedächtnisses gespreizt zu werden.

3. Vorrichtung nach Anspruch 1,
**dadurch gekennzeichnet,**
**dass** die andere Verankerungszone in ihrer Dicke eine Öffnung (Ib1) aufweist, um unter der Wirkung des Formgedächtnisses eine elastische Verformung zu ermöglichen.

4. Vorrichtung nach einem beliebigen der vorhergehenden Ansprüche,
**dadurch gekennzeichnet,**
**dass** der Körper einen abgeflachten Querschnitt aufweist.

5. Vorrichtung nach einem beliebigen der vorhergehenden Ansprüche,
**dadurch gekennzeichnet,**
**dass** eine der Verankerungszonen (1a) einen Schenkel oder Stiel aufweist, der geeignet ist, unter der Wirkung des Formgedächtnisses umgebogen zu werden.

6. Vorrichtung nach einem beliebigen der vorhergehenden Ansprüche,
**dadurch gekennzeichnet,**
**dass** die mittige Zone (1c) in Draufsicht insgesamt im Wesentlichen dreieckig ausgebildet ist.

7. Vorrichtung nach einem beliebigen der vorhergehenden Ansprüche,
**dadurch gekennzeichnet,**
**dass** die Länge der mittigen Zone (1c) zwischen 3 mm und 5 mm liegt, wodurch sie geeignet ist, eine leichte Verschiebung bei der Zentrierung der Vorrichtung zu erlauben.

8. Vorrichtung nach einem beliebigen der vorhergehenden Ansprüche,
**dadurch gekennzeichnet,**
**dass** die Verbindungszone (1d) in Draufsicht insgesamt im Wesentlichen rechteckig ausgebildet ist.

9. Vorrichtung nach einem beliebigen der vorhergehenden Ansprüche,
**dadurch gekennzeichnet,**
**dass** die Verbindungszone (1d) geschlitzt ist, damit die Wirkung der Aufweitung durch Formgedächtnis und der Verstärkung der Verankerung in dem Diaphysenbereich ihre Vorteile entfaltet.

10. Vorrichtung nach einem beliebigen der vorhergehenden Ansprüche,
**dadurch gekennzeichnet,**
**dass** die Schenkel an einer ihrer Seiten Rasten aufweisen.

11. Vorrichtung nach einem beliebigen der vorhergehenden Ansprüche,
**dadurch gekennzeichnet,**
**dass** die Schenkel vor der Implantation gerade sind und so angeordnet sind, dass sie nach Inanspruchnahme des Formgedächtnisses gewellt werden, um eine Verkürzung zu ermöglichen.
